(19) Europäisches Patentamt
European Patent Office
Office européen des brevets

(11) **EP 4 140 450 A1**

(12) **EUROPEAN PATENT APPLICATION**

(43) Date of publication:
**01.03.2023 Bulletin 2023/09**

(21) Application number: **22189851.3**

(22) Date of filing: **11.08.2022**

(51) International Patent Classification (IPC):
***A61F 2/26*** (2006.01)

(52) Cooperative Patent Classification (CPC):
**A61F 2/26**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **12.08.2021 US 202163232216 P**

(71) Applicant: **Coloplast A/S
3050 Humlebaek (DK)**

(72) Inventors:
• **ALLEN, John J.
Mendota Heights, 55118 (US)**
• **LENZ, Megan
Lino Lakes, MN 55014 (US)**

Remarks:
The references to the drawing(s) no. 1C are deemed
to be deleted (Rule 56(4)(6) EPC).

(54) **AN INFLATABLE BLADDER FOR A PENILE PROSTHESIS**

(57) An inflatable bladder (60) of a penile prosthesis (26) has a multi-layered wall structure with a wall spacing between walls to allow an interior distal wall end (86) to move independently of an exterior distal wall end. (96)

Fig. 5

EP 4 140 450 A1

**Description**

**Background**

Inflatable penile prosthetics have proven useful in treating erectile dysfunction. The typical inflatable prosthetic has a pair of prostheses implanted in the penis, a reservoir implanted in the abdomen, and a pump implanted in the scrotum to move liquid out from the reservoir and into the prostheses. A natal male has two corpora cavernosa in the penis that are separated by a septum, and each corpus cavernosum receives one prosthesis (or, inflatable bladder, also called a cylinder). The movement of the liquid out from the reservoir and into the cylinder inflates the prosthetic to achieve an erect penis, and the movement of the liquid out from the cylinder and back to the reservoir deflates the prosthetic to achieve a flaccid penis. The user desires the appearance and the function of the prosthetic to mirror the appearance and function of a natal penis in both the erect and flaccid states.

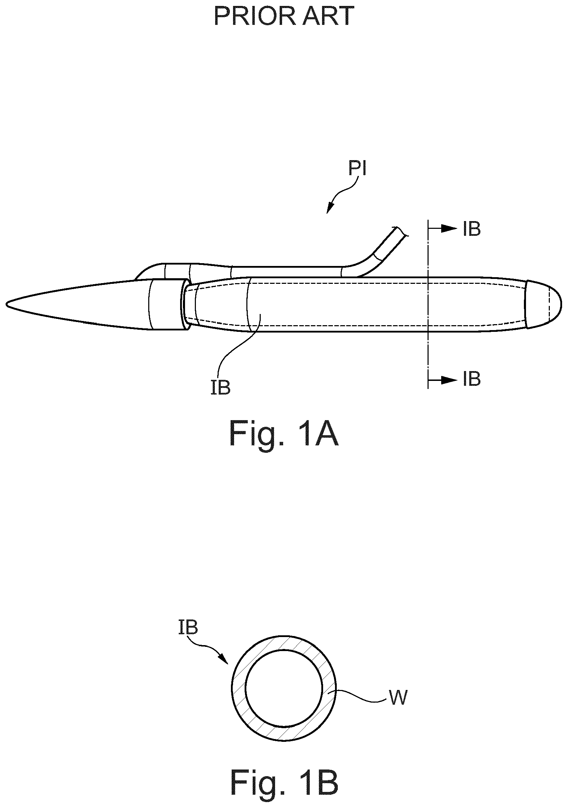

Fig. 1A is a front view of a prior art penile prosthesis P1, and Fig. 1B is a cross-sectional view of the prior art penile prosthesis P 1 having an inflatable bladder IB formed of a single wall W of elastic material having a wall thickness in a range from about 0.40 mm to 0.51 mm (0.016 inches to 0.020 inches). Other prior art penile prostheses have an inflatable bladder with a wall formed of a laminate of layers that are constrained at least along the proximal end portion and the distal end portion of the bladder.

The prior art inflatable bladder with the single wall W of elastic material, when compared to the inflatable bladder with the laminate of layers, has improved toughness that supports inflation to higher pressures, which provides the user with an improved erection when inflated. The prior art inflatable bladder with the laminate of layers, when compared to the inflatable bladder IB of Fig. 1B, has improved bending flexibility when flaccid due to its layers being formed from a softer, more flexible polymer, but the price of the more flexible polymer selection is a limited maximum inflation pressure and thus a less rigid erection. Both forms of prior art inflatable bladders exhibit a level of "dog-eared" DE corners, although an inflatable bladder formed of a soft material will generally have a less noticeable formation of dog-ears than an inflatable bladder formed of stiffer material.

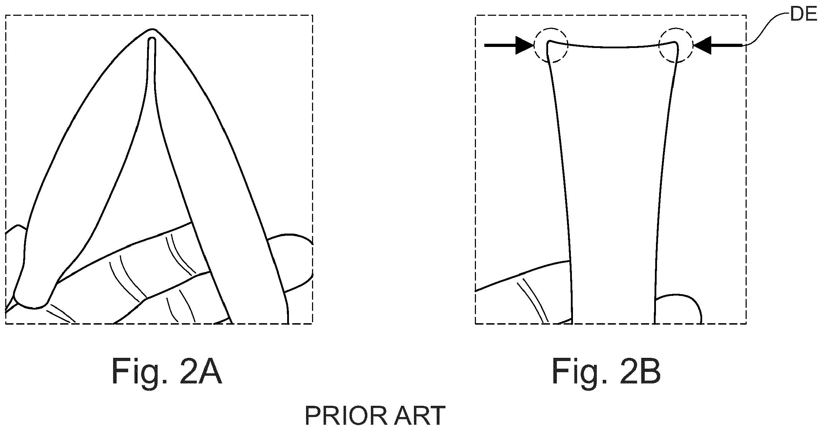

Fig. 2A shows the single wall inflatable bladder IB in a deflated state and representing a flaccid condition of the penis when bent along its long axis. Fig. 2B shows the presence of dog-ears at corners for the bent and deflated bladder IB. The dog-ears are formed when the walls of the deflated cylinder collapse and come together at the two opposed lateral corners. The folded layers of the cylinder wall present sharp corners that can be felt by a user. Dog-ears are undesirable and can be perceived as uncomfortable by the user.

Improvements to inflatable penile prosthetics would be welcomed by both patients and surgeons.

**Summary**

The inflatable bladder (or cylinder) of one of the market-leading implantable penile prostheses (IPP) has a solid bladder wall formed of a urethane-based polymeric material. The urethane-based polymeric material has exceptional toughness, allowing inflation of the cylinder to higher pressures and larger diameters compared to competitive IPP cylinders, which provides the user with best-in-class girth and rigidity of erection. However, user feedback suggests that the cylinder flexibility in the flaccid state might be lacking. Flexibility is analogous to how easily a deflated cylinder can be squeezed or compressed in the radial direction, which is readily apparent when a flaccid implant is bent along its long axis and a compressive force is applied in a radial (or lateral) direction at the bend. A lack of radial flexibility is associated with a higher perceived stiffness and the formation of "dog-ears" or sharp corners when a deflated cylinder (in a flaccid penis) is bent on its long axis.

This disclosure provides an inflatable bladder with improved flexibility in the deflated state with excellent toughness and tensile strength present during the inflated state. The disclosed inflatable bladders provide a solution to the dog-ears problem by reducing or eliminating the presence of dog-eared corners in deflated bladders. In general, embodiments describe a bladder wall having multiple layers and a spacing between the layers to increase the flexibility of the cylinder when flaccid while retaining toughness, rigidity and tensile strength when erect. The disclosed inflatable bladders, when flaccid, have walls that are able to slide past each other thereby making the flaccid bladder very flexible. However, when erect, the gaps between the cylinder walls and distal end are reduced or eliminated due to inflation of the bladder and the walls of the bladder move together; therefore, the multi-layered bladder walls behave more like a single, thicker wall cylinder.

A bladder wall having multiple layers and a spacing between the layers provides the inflatable bladder, when flaccid, with reduced severity of the dog-ear effect and can reduce the occurrence of the dog-ear effect, both of which are desirable outcomes and advantageous to the user. A bladder wall having multiple layers where the interior wall layer is of the disclosed "wavy" cylinder style provides the inflatable bladder, when flaccid, with both the reduced severity of the dog-ear effect and increases a radius of the bent portion of the deflated and flaccid bladder, which reduces beneficially and advantageously the perception (the user's sense of feeling the dog-ear).

One embodiment provides an inflatable bladder of a penile prosthesis having a plurality of nested cylinders with a second wall spaced away from a first wall by a gap distance to allow at least the second wall of a second cylinder to move independently of the first wall of a first cylinder. Embodiments of the inflatable bladder desirably allow the walls of the nested cylinders to move independently whether the prosthesis is flaccid (uninflated) or rigid (inflated).

One embodiment provides an inflatable bladder comprising a first cylinder nested inside a second cylinder, or "nested cylinders." The inflatable bladder of nested cylinders has two or more walls, where the sum of the individual wall thicknesses is approximately equal to the total thickness of a conventional cylinder having a single bladder wall.

Nested or nested inside means a first cylinder is disposed or placed inside a second cylinder with a spacing between the cylinders to allow the distal ends of the cylinders to be unrestrained and move independently of each other. Nested inside includes a first cylinder is disposed or placed inside a second cylinder and the walls and the distal ends of the cylinders are unrestrained and move independently of each other. Nested inside includes that the first cylinder is an interior cylinder, and the second cylinder is an exterior cylinder of the inflatable bladder. Nested inside includes a first cylinder is disposed or placed inside a second cylinder with a spacing between the cylinders, where the proximal ends of the cylinders (the rear or backend portions) are of equal length. Nested inside includes a first cylinder is disposed or placed inside a second cylinder, where a first proximal portion of the first cylinder is connected to the second proximal portion of the second cylinder 90, and alternatively, includes where an intermediate layer is placed between the first proximal portion of the first cylinder and the second proximal portion of the second cylinder 90. Nested inside includes embodiments where the first proximal portion of the first cylinder (the inside or interior cylinder) is connected to the rear tip, and alternatively, where the second proximal portion of the second cylinder (the outside or exterior cylinder) is connected to the rear tip. Nested inside includes where pair of nested cylinders are connected at their respective proximal end portions, and both respective proximal end portions of the nested cylinders are bonded or permanently connected with the rear tip.

The basic mechanics underlying the concept is the bending behavior of material related to variations in thicknesses. A simple beam with a rectangular cross-section has a bending stiffness that is proportional to the cube of its thickness (the dimension perpendicular to the bend axis). Qualitatively, a prothesis bladder wall, when deflated and flattened, approximates the rectangular cross-sectional shape. Thus, it is reasonable to approximate the bending stiffness E as proportional to the cube of the bladder wall thickness T:

$$E \propto T^3$$

We have postulated and shown by measurements of working examples that a cylinder wall formed to have multiple unconstrained layers will have a reduced bending stiffness and a significantly reduced occurrence of dog-ears. As one example, a cylinder wall formed to have four (4) unconstrained layers, where the four layers are selected to have a combined thickness approximately equal to the thickness of a conventional single layer bladder wall, the bending stiffness for the nested cylinder walls becomes considerably less:

$$E \propto 4*(T/4)^3 = T^3/16$$

For example, a bladder having two nested cylinder walls will have a bend stiffness that is 4X less than a bladder wall with a solid wall of the same total thickness; a bladder having three nested cylinder walls will have a bend stiffness that is 9X less than a bladder wall with a solid wall of the same total thickness; and, as shown in the example formula above, a bladder having four nested cylinder walls will have a bend stiffness that is 16X less than a bladder wall with a solid wall of the same total thickness. The reduced bend stiffness for nested bladder walls provides a more comfortable penile implant when flaccid while still having the best-in-class girth and rigidity when erect.

Tensile stiffness of the inflatable bladder, which is directly proportional to the total wall thickness, remains unchanged. Thus, it is possible to independently control tensile stiffness and bend stiffness since the layers of the inflatable bladder are allowed to slide relative to each other in the bending region. Alternatively, it is possible to increase tensile strength, if desired, and keep bend stiffness the same. It is possible to further tune the bend properties of embodiments of an inflatable bladder having multiple walls by selecting different thicknesses for each wall layer in the nested cylinders of the inflatable bladder.

An inflatable bladder of a penile prosthesis has a multi-layered wall structure with a wall spacing between walls to allow an interior distal wall end to move independently of an exterior distal wall end

One embodiment provides a penile prosthesis comprising:

a rear tip, with a lumen formed inside of the rear tip, and tubing connected to the rear tip and communicating with the lumen; and

an inflatable bladder coupled to the rear tip, the inflatable bladder comprising:

a first cylinder nested inside a second cylinder, the first cylinder comprising a first proximal portion, a first distal end portion that terminates in a first distal end, and a first wall extending between the first proximal portion and the first distal end portion,
the second cylinder comprising a second proximal portion, a second distal end portion that terminates in a second distal end, and a second wall extending between the second proximal portion and the second distal end portion;

wherein the first proximal portion of the first cylinder is connected to the rear tip;
wherein an interior of the first cylinder provides the inflatable bladder with an inflation volume communicating with the lumen in the rear tip;
wherein the first distal end is spaced away from the second distal end by a gap distance allowing the first distal end to move independently of the second distal end.

The advantages provided by the penile prosthesis is that the bend stiffness for this two-wall inflatable bladder is lower than the bend stiffness of an inflatable bladder having a comparable total wall thickness, and the reduced bend stiffness of the nested cylinders reduces or eliminates the presence of dog-ears for deflated bladders that become bent during use. Another advantage is that at least the distal ends of the two cylinders are allowed to move independently, which reduces dog-ear compressive stiffness. In some examples, the distal ends and the first and second walls of the two nested cylinders move independently, sliding relative to each other, and this has been evaluated to reduce bending stiffness for an inflatable bladder and reduce or eliminate the presence of dog-ears.

One aspect of the embodiment includes the first proximal portion of the first cylinder is connected to the second proximal portion of the second cylinder, and the second proximal portion of the second cylinder is connected to the rear tip. The advantage of this nested-and-bonded approach to the proximal ends is ease of manufacturing.

One aspect of the embodiment includes the second wall is spaced away from the first wall by a gap distance allowing the first distal end to move independently of the second distal end. A wall spacing that allows distal end spacing also reduces dog-ear bend stiffness and should improve patient comfort.

One aspect of the embodiment includes the first distal end of the first cylinder is spaced by the gap distance away from the second distal end of the second cylinder. It can be an advantage to have the distal ends of the nested cylinders spaced apart by the same gap distance between the walls to encourage independent movement between the distal ends. Alternatively, it can be an advantage to have the distal ends of the nested cylinders spaced apart by a gap distance that is different from the gap distance between the walls, so long as the cylinders are allowed to have independent movement between at least the wall portion where a bend would be expected to occur.

One aspect of the embodiment includes an entirety of a perimeter of the first proximal portion of the first cylinder is sealed to the second proximal portion of the second cylinder. The proximal portions of the cylinders are coupled to the rear tip, and it can be an advantage to seal off the first cylinder from the second cylinder so only the first cylinder is inflated with liquid.

One aspect of the embodiment includes the first proximal portion of the first cylinder is sealed to both the second proximal portion of the second cylinder and to the rear tip. It can be an advantage to secure the first cylinder to the rear tip to ensure inflation of the first cylinder without liquid leakage, and to seal the first cylinder to the second cylinder to provide a robust connection between the three parts that form the proximal end of the prosthesis.

One aspect of the embodiment further includes a third cylinder inserted between the first cylinder and the second cylinder, with a proximal portion of the third cylinder sealed between the first proximal portion of the first cylinder and the second proximal portion of the second cylinder. The advantage of this aspect is that a bladder having three nested cylinder walls will have a bend stiffness that is calculated to be about 9X less than a bladder wall with a solid wall of the same total thickness

One aspect of the embodiment further includes a spacing material inserted into the gap distance and adapted to separate the second wall and the first wall. The advantage of a spacing material between adjacent walls of nested cylinders is to ensure a physical separation between at least the walls to allow the cylinders to move independently when deflated, which ensures that the wall portions will slip or slide one relative to the other(s). this unconstrained movement has been shown to reduce or eliminate the formation of dog-ears in a deflated prosthesis when the bladder portion is bent.

One aspect of the embodiment further includes a spacing material inserted into the gap distance, where the spacing material prevents adhesion between the second wall and the first wall. The advantage of a material that prevents adhesion between adjacent layers is to ensure relative movement or slippage between those layers by limiting sticking between the layers.

One aspect of the embodiment further includes a spacing material inserted into the gap distance, where the spacing material is a woven textile, a knitted textile, a wax, a foam, a film, a gel, or a silicone liquid. These materials are expected to be compatible with manufacturing and also serve to physically separate at least the wall portions of two or more nested cylinders of an inflatable bladder, which should reduce the bending stiffness and reduce or eliminate the occurrence of dog-ears in a deflated bladder that is bent.

One aspect of the embodiment further includes a low adhesion surface coating applied to the first wall and adapted to allow the first wall to slide relative to the second wall. The advantage is that the coating does not need to remain and physically separate the layers. The coating would ideally prevent adhesion between the layers but not constrain the layers in the shear direction, thus allowing the layers to slide relative to each other. The separation coating would have a low shear stiffness, or simply break apart or degrade after the cylinders' layers are nested. For example, the separation coating might be a wax-like film that melts near or below body temperature but remains solid during the manufacturing process. The separation layers could also be extracted or removed by melting or vaporizing with a higher temperature treatment. The separation coating might be low density elastomer foam (e.g., polyurethane, silicone, polyethylene), or a low shear gel.

One aspect of the embodiment includes each of the first cylinder and the second cylinder is formed of a first polymer, and further comprising a second polymer different from the first polymer, with the second polymer coated onto the first wall of the first cylinder and adapted to separate the second wall and the first wall. The advantage is ease of manufacturing by dip-coating, for example, a different polymer onto the first cylinder, where the different polymer serves as a separation layer.

Another aspect includes a liquid located between the second polymer and the second wall of the second cylinder. The advantage is that a liquid separation layer will ensure that the layers in the nested cylinder(s) are able to move independent one from the other.

One aspect of the embodiment further includes an annular sleeve coupled to the first wall of the first cylinder and adapted to separate the second wall and the first wall. The advantage is ease of manufacturing by placing the annular sleeve on the first cylinder, where the annular sleeve serves as a separation layer.

Another aspect includes the annular sleeve has a sleeve length and a sleeve diameter, where the sleeve length is less than a length of the first wall measured between the first proximal portion and the first distal end portion. The advantage is the ease of placement of the annular sleeve along a relatively straight wall section, which facilitate manufacturing while providing an effective separation between adjacent walls of nested cylinder(s).

One aspect of the embodiment further includes filament wrapped multiple revolutions around a circumference of the first wall of the first cylinder and adapted to separate the second wall and the first wall. The advantage is ease of manufacturing by placing the filament around the first cylinder, where the filament serves as a separation layer.

One aspect of the embodiment includes the first wall of the first cylinder comprises a series of troughs formed into the first wall and aligned longitudinally along the first wall. The advantage is that the separation portion can be formed directly into one of the cylinders to provide and ensure separation between at least the walls of the nested cylinder(s).

Another aspect includes the series of troughs approximate a sine wave having a peak formed by an exterior surface of the first wall located between adjacent troughs. The advantage is an undulating structure where the wall thickness of the undulations is about constant, and the amplitude of the wall undulation provides the gap distance of the separation.

Another embodiment provides an inflatable bladder of a penile prosthesis, the inflatable bladder comprising:

　　a first cylinder adapted to be inflated by liquid and comprising a first proximal portion, a first distal end portion that terminates in a first distal end, and a first wall extending between the first proximal portion and the first distal end portion; and
　　a second cylinder comprising a second proximal portion, a second distal end portion that terminates in a second distal end, and a second wall extending between the second proximal portion and the second distal end portion;
　　wherein an interior of the first cylinder provides the inflatable bladder with an inflation volume adapted to provide the inflatable bladder with an erect state;

wherein the second wall is spaced away from the first wall by a gap distance allowing at least the second wall of the second cylinder to move independently of the first wall of the first cylinder.

The advantages provided by this inflatable bladder is a lower bend stiffness than the bend stiffness of an inflatable bladder having a comparable total wall thickness, and the reduced bend stiffness of the nested cylinders reduces or eliminates the presence of dog-ears for deflated bladders that become bent during use. Another advantage is that at least the distal ends of the two cylinders are allowed to move independently, which reduces bend stiffness. In some examples, the distal ends and the first and second walls of the two nested cylinders move independently, sliding relative to each other, and this has been evaluated to reduce bending stiffness for an inflatable bladder and reduce or eliminate the presence of dog-ears.

## Brief Description of the Drawings

The accompanying drawings are included to provide a further understanding of embodiments and are incorporated in and constitute a part of this specification. The drawings illustrate embodiments and together with the description explain principles of embodiments. Other embodiments and advantages of embodiments will be readily appreciated as they become better understood by reference to the following detailed description. The elements of the drawings are not necessarily to scale relative to each other. Like reference numerals designate corresponding similar parts.

Fig. 1A is a perspective view of a prior art penile prosthesis.
Fig. 1B is a cross-sectional view of the prior art penile prosthesis having an inflatable bladder formed of a single wall of plastic material.
Fig. 1C is a cross-sectional view of another prior art penile prosthesis having an inflatable bladder formed of a laminate of bonded layers.
Fig. 2A and Fig. 2B illustrate the inflatable bladder of the prior art exhibiting the undesirable dog ear effect when in a bent position.
Fig. 3 is a perspective view of one embodiment of an implant including a pair of penile prostheses.
Fig. 4 is a perspective view of one embodiment of a penile prosthesis.
Fig. 5 is an exploded view of the penile prosthesis of Fig. 4 showing the inflatable bladder separated from the rear tip.
Fig. 6 is a cross-sectional view of the inflatable bladder of Fig. 5.
Fig. 7 is a series of partial cross-sectional views of the inflatable bladder of Fig. 6.
Fig. 8A and Fig. 8B are cross-sectional views of one embodiment of an inflatable bladder having two separated walls.
Fig. 9A and Fig. 9B are cross-sectional views of one embodiment of an inflatable bladder having three separated walls.
Fig. 10A and Fig. 10B are cross-sectional views of one embodiment of an inflatable bladder having four separated walls.
Fig. 11 is a series of partial cross-sectional views of one embodiment of an inflatable bladder.
Fig. 12 is a series of partial cross-sectional views of one embodiment of an inflatable bladder.
Fig. 13 is a perspective view of one embodiment of an inner cylinder of an inflatable bladder with the inner cylinder including an annular sleeve.
Fig. 14 is a perspective view of one embodiment of an inner cylinder of an inflatable bladder with the inner cylinder including a filament wrapped thereabout.
Fig. 15 is an exploded view of a first cylinder removed from the second cylinder and showing a series of partial cross-sectional views along one embodiment of an inflatable bladder.
Fig. 16A is a side view of the first cylinder of Fig. 15 showing a series of troughs formed longitudinally in the first wall.
Fig. 16B is a perspective view of a distal end portion of the first cylinder of Fig. 16A.
Fig. 16C is a distal end view of a distal end of the first cylinder of Fig. 16A.
Fig. 17 is a perspective view of the distal end portion of an assembled inflatable bladder of Fig. 15.
Fig. 18A is a top view of an inflatable bladder inserted into a test apparatus.
Fig. 18B is a side view of an inflatable bladder in the test apparatus of Fig. 18A.
Fig. 19 is a graph of comparative buckling force results for embodiments of inflatable bladders across a range of fill volume percentage.

## Detailed Description

In the following Detailed Description, reference is made to the accompanying drawings, which form a part hereof, and in which is shown by way of illustration specific embodiments in which the invention may be practiced. In this regard, directional terminology, such as "top," "bottom," "front," "back," "leading," "trailing," etc., is used regarding the orientation of the Fig.(s) being described. Because components of embodiments can be positioned in several different orientations, the directional terminology is used for purposes of illustration and is in no way limiting. It is to be understood that other embodiments may be utilized and structural or logical changes may be made without departing from the scope of the present invention. The following detailed description, therefore, is not to be taken in a limiting sense, and the scope of the present invention is defined by the attached claims.

The features of the various exemplary embodiments described in this application may be combined with each other, unless specifically noted otherwise.

The term "proximal" in this application means that part that is situated next to or near the point of attachment or origin or a central point; for example, as located toward a center of the human body. The portion of an implant that is closest to a center of a patient's body is the proximal portion of the implant. For an implanted cylinder of an inflatable penile prosthetic, the end of the cylinder located in the crus penis is a proximal end of the cylinder.

The term "distal" in this application means that part that is situated away from the point of attachment or origin or the central point; for example, as located away from the center of the human body. For an implanted cylinder of an inflatable penile prosthetic, the end of the cylinder located in the glans penis is a distal end of the cylinder.

End means endmost. A distal end is the furthest endmost location of a distal portion of a thing being described, whereas a proximal end is the nearest endmost location of a proximal portion of the thing being described. The portion next to or adjacent to an end is an end portion. For example, a 12-inch ruler has a center at 6 inches, a first end at zero inches and a second, opposite end at 12 inches, a first end portion adjacent to the first end and a second end portion adjacent to the second end.

A penile prosthesis is a device that is suited for implantation into the body. When implanted, the prosthesis is called a prosthetic.

An implanted penile prosthetic has proven useful in treating erectile dysfunction. One acceptable implanted penile prosthetic includes two inflatable cylinders implanted in the penis, a pump implanted in the scrotum or other internal space of the body, and a liquid holding reservoir implanted in the abdomen or other internal space of the body, with the pump connected to the cylinders and the reservoir.

In an implantation procedure, the penis of the patient is incised in a corporotomy to expose the two corpora cavernosa that are aligned axially in a side-by-side orientation within the penis. A cutting implement, such as a curved Mayo scissors, is employed to penetrate the fascia of the penis and form an opening accessing each corpus cavernosum. Subsequently, each corpus cavernosum is dilated (opened) with an appropriate dilation tool to form a recess that is sized to receive one of the two cylinders of the penile prosthesis.

Thereafter, a tool (referred to by surgical practitioners as a "Furlow" introducer) is inserted into each dilated corpus cavernosum to measure a length of the penis distally and proximally to determine a desired length of the cylinders to be implanted. A prosthesis of the appropriately selected length is secured to a suture, and the suture is secured to a needle (sometimes called a "Keith" needle). The Keith needle is attached to the Furlow introducer. The surgeon inserts the Furlow introducer into the dilated corpus cavernosum. The surgeon steadies the Furlow introducer with one hand and pushes a plunger (or obturator) of the Furlow introducer with the other hand. Pushing the plunger pushes the Keith needle out of the introducer, through tissue of the penis, and out the glans penis. The exposed portion of the needle is handled by the surgeon, removed from the suture, and discarded. The portion of the suture extending from the glans penis is subsequently employed to tow the prosthesis into place within the dilated corpus cavernosum.

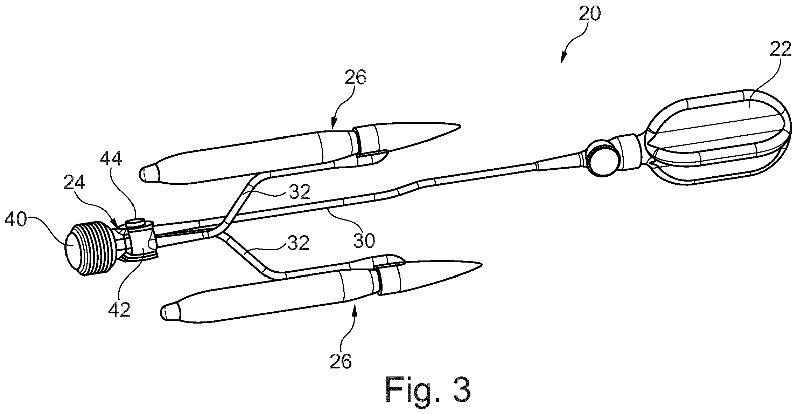

Fig. 3 is a perspective view of one embodiment of an implantable medical device 20 including a penile prosthesis 26. The implantable medical device 20 includes a reservoir 22 sized to contain a volume of liquid, a pump 24 connected to the reservoir 22 and operable to move the liquid out from the reservoir 22, and a pair of penile prostheses 26.

The reservoir 22 is attachable intra-operatively to the pump 24 by tubing 30, and the pump 24 is attachable intra-operatively to each penile prosthesis 26 by separate tubing 32.

The reservoir 22 is sized for implantation within the human body, for example within the abdomen. The reservoir 22 is sized to retain a volume of liquid useful in inflating the penile prostheses 26, for example with a volume in a range from 50-350 cc. One useful size of reservoir 22 contains about 200 mL of liquid. The liquid is aqueous, for example saline. Suitable materials for fabricating the reservoir 22 include silicone, polymers such as urethanes, a blend of polymers with urethane, copolymers of urethane, or the like. In one exemplary fabrication process, one of the suitable materials identified above is molded into a container shape appropriate for implantation in the space of Retzius or in the abdomen.

The pump 24 includes a pump bulb 40 connected to a pump housing 42. The pump bulb 40 is pliant and configured to be repeatedly squeezed by the user of the medical device 20 to move liquid into the penile prostheses 26. The pump housing 42 contains the valving that operates to allow a flow of liquid from the reservoir 22 to the penile prostheses 26 in response to a squeezing of the pump bulb 40, and a flow of liquid out of the penile prostheses 26 back to the reservoir 22 in response to activation of a deflation button 44.

The components of the medical device 20 (the reservoir 22, the pump 24, the penile prostheses 26, and the tubing 30, 32) are generally provided unassembled in a kit of parts along with instructions for use. The components are assembled immediately prior to surgery, or intra-operatively, as determined by the surgeon. For example, each of the tubing lengths 30, 32 have a portion that is attached to the reservoir 22 and a portion that is attached to the penile prostheses 26, respectively, and a portion that is secured to the pump 24. The tubing lengths are connected intra-operatively with some form of tubing connector useful with surgical implants. The components of the medical device 20 are illustrated in an assembled configuration in Fig. 3.

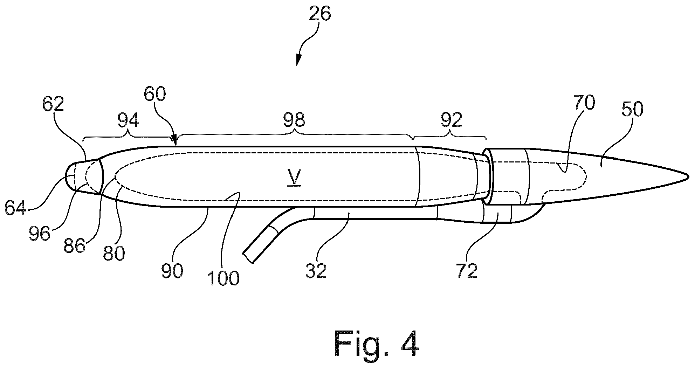

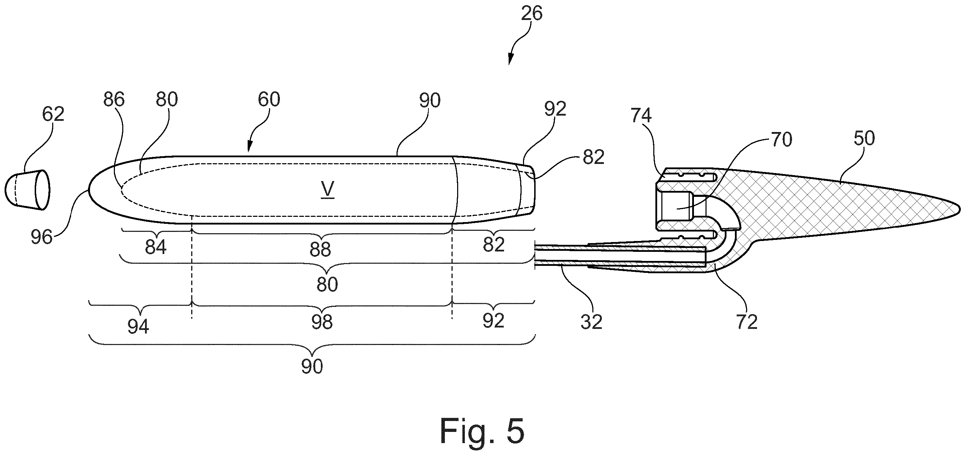

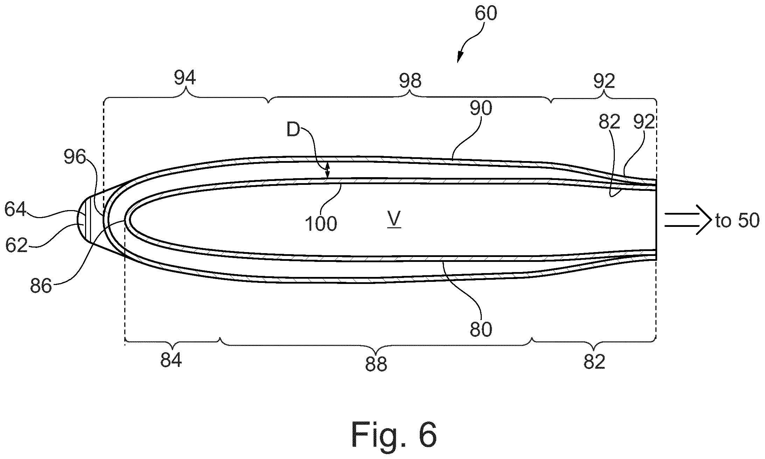

Fig. 4 is a perspective view of one embodiment of one penile prosthesis 26 of the pair of penile prostheses, Fig. 5 is an exploded view of the penile prosthesis 26, and Fig. 6 is a cross-sectional view of an inflatable bladder of the penile prosthesis 26.

The penile prosthesis 26 includes two nested cylinders having thinner, spaced-apart walls, where the spaced walls allow each cylinder to move independently when the penile prosthesis 26 is deflated which results in a significant reduction in the occurrence of dog-ears when the deflated prosthesis is bent. When the deflated prosthesis 26 is bent or folded over by the user, the corner area of the bent / folded prosthesis may still be present, however, the stiffness of the bend of folded area is greatly reduced. Consequently, the undesirable dog-ear affect (as measured by the buckling strength) is also greatly reduced, resulting in improved user comfort.

The penile prosthesis 26 includes a rear tip 50 connected to an inflatable bladder 60. An end tip 62 is optionally coupled to the inflatable bladder 60, where the end tip 62 includes a suture channel 64 that is useful when towing the penile prosthesis into the dilated corpus cavernosum.

The rear tip 50 forms a lumen 70 communicating with a strain relief section 72 that is attachable to the tubing 32. A slot 74 is formed in the rear tip 50 that is sized to receive and bond with the inflatable bladder 60. The liquid from the reservoir 22 (Fig. 3) moves through the tubing 32, the strain relief section 72, and through the lumen 70 in the rear tip 50 for inflation of the inflatable bladder 60.

The inflatable bladder 60 includes a first cylinder 80 nested inside a second cylinder 90 with a spacing between the cylinders 80, 90 to allow the walls and the distal ends of the cylinders 80, 90 to be unrestrained and move independently of each other. The nested orientation means that the first cylinder 80 is an interior cylinder and the second cylinder 90 is an exterior cylinder of the inflatable bladder 60. The end tip 62, if employed, is connected to the second (or exterior) cylinder 90 of the inflatable bladder 60.

The first cylinder 80 includes a first proximal portion 82, a first distal end portion 84 that terminates in a first distal end 86, and a first wall 88 extending between the first proximal portion 82 and the first distal end portion 84.

The second cylinder 90 includes a second proximal portion 92, a second distal end portion 94 that terminates in a second distal end 96, and a second wall 98 extending between the second proximal portion 92 and the second distal end portion 94.

Figs. 5 and 6 illustrate embodiments where the first proximal portion 82 of the first cylinder 80 is connected to the second proximal portion 92 of the second cylinder 90, and Fig. 4 illustrates the second proximal portion 92 of the second cylinder 90 is connected to the rear tip 50. The pair of nested cylinders 80, 90 are thus connected at their respective proximal end portions 82, 92, and at least the proximal end portion 92 of the second cylinder 90 is bonded or permanently connected within the slot 74 formed in the rear tip 50 (Fig. 5).

In embodiments, the first proximal portion 82 of the first cylinder 80 is bonded or physically connected to the second proximal portion 92 of the second cylinder 90. In embodiments, an inner face of the first proximal portion 82 of the first cylinder 80 is bonded or physically connected to the rear tip 50. Generally, the first proximal portion 82 of the first cylinder 80 will touch or be immediately adjacent to the second proximal portion 92 of the second cylinder 90. Acceptable methods of bonding the first proximal portion 82 of the first cylinder 80 to the second proximal portion 92 of the second cylinder 90 include adhesive bonding, or solvent bonding, ultrasonic welding, or heat lamination.

When assembled, an interior 100 or an interior surface 100 of the first cylinder 80 provides the inflatable bladder 60 with an inflation volume V communicating with the lumen 70 of the rear tip 50. Liquid delivered into the volume V of the first cylinder 80 will enlarge the bladder 60 by expansion of the first cylinder 80. The second cylinder 90 will be expanded but is free to move or slide relative to the first cylinder 80. The separate thin walls 88, 98 of each cylinder 80, 90 are free to move independently from each-other in the flaccid state. However, when inflated, the inflatable bladder will present an approximately same or similar wall thickness of a typical single wall thickness inflatable bladder, and thus maintain the desired nominal tensile/performance properties of current prosthesis cylinders

The second wall 98 is spaced away from the first wall 88 by a gap distance D allowing the first distal end 86 to move independently of the second distal end 96. The gap distance D allows the wall 88 of the first cylinder 80 to move independently of the wall 98 of the second cylinder 90 so that the walls 88, 98 and the ends 86, 96 are unconstrained one from another and able to move independently of each other in a shear direction. The gap distance D reduces or prevents the walls 88, 98 from sticking to each other, and the unrestrained freedom of movement of the cylinders 80, 90 has been measured to significantly reduce the dog-ear corners when the deflated bladder 60 is bent.

In one embodiment, the assembled inflatable bladder 60 has the first proximal portion 82 of the first cylinder 80 bonded or physically connected within the slot 74 in the rear tip 50 (Fig. 5) and the second cylinder 90 is disposed over and nested with the first cylinder 80 and not bonded to the rear tip 50 or the first cylinder 80. The first cylinder 80 is thus connected to the rear tip and communicates with the lumen 70 to allow the first cylinder 80 of the inflatable bladder 60 to be inflated to the erect state. The second (or outside) cylinder 90 is fully unconstrained relative to the first cylinder 80 and the rear tip 50 to move and flex as the flaccid bladder moves and is flexed. The tapered geometry of the first proximal portion 82 of the first cylinder 80 prevents the second cylinder 90 from sliding off the cylinder 80. In addition, when implanted, the inflatable bladder 60 is maintained within the tunica of the penis, which prevents separation of the two cylinders 80, 90.

The nested cylinders may be formed in a variety of methods. Cylinders may be fabricated by molding followed by inserting one molded cylinder into another. Cylinders may be dip coated onto a mandrel, followed by removal of the cylinder from the mandrel and inserting a dip coated cylinder into another cylinder. The dip coating approach also allows another material to be dip coated onto a surface of a cylinder during the dipping process, for example by dip coating a spacer material onto a cylinder before inserting it into a second, outer cylinder. Several suitable approaches are available for the formation of hollow cylinders that are subsequently nested into an inflatable bladder.

Several embodiments of bladder constructions are described below that provide for the independent movement between a pair of nested cylinders in an inflatable bladder.

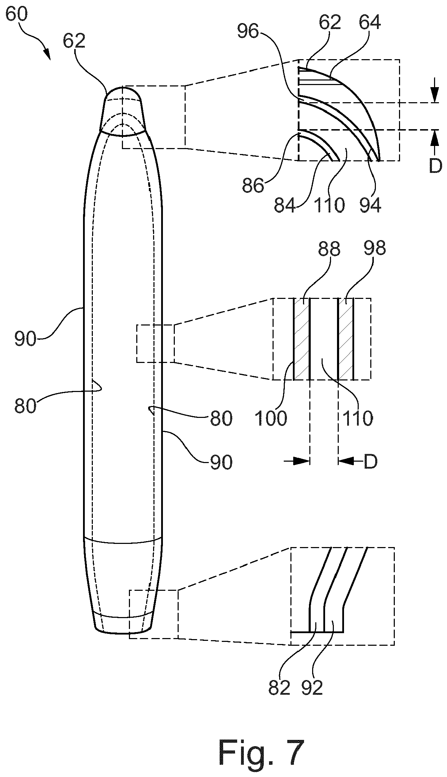

Fig. 7 is a series of partial cross-sectional views of the inflatable bladder 60 with the two nested cylinders 80, 90 illustrating one option for coupling the cylinders 80, 90. The embodiment illustrated in Fig. 7 shows two nested cylinders 80, 90 with a spacing between the walls 88, 98 and the distal ends 86, 96. The spacing provides a void space that is filled with air, for example, between portions of the cylinders 80, 90 to facilitate independent movement between the walls 88, 98 and the distal ends 86, 96 of the cylinders 80, 90. The independent movement between cylinders increases the flexibility of the prosthesis, which is particularly noticeable and useful when the prosthesis 26 is deflated.

The first cylinder 80 is nested inside of the second cylinder 90 and the first proximal portion 82 of the first cylinder 80 is connected to the second proximal portion 92 of the second cylinder. Thus, the remaining portions of the cylinders 80, 90, notably the walls 88, 98 and the distal end portions 84, 94 are not coupled together and are free to move one relative to the other in an unrestrained manner.

In one embodiment, the first distal end 86 of the first cylinder 80 is spaced by the gap distance D away from the second distal end 96 of the second cylinder 90. That is to say, the gap distance D at the ends 86, 96 is the same as the gap distance D between the walls 88, 98. In this embodiment, the gap space D is a void space 110 that is filled with air, or a space which eventually becomes occupied by air. In other embodiments, the gap space D (void space 110) is filled with saline or a gel to provide the inflatable bladder 60 with a partially filled conformation, giving the user the desirable sensation of a softer, more flexible bladder.

In one embodiment, an entirety of a perimeter of the first proximal portion 82 of the first cylinder 80 is sealed to the second proximal portion 92 of the second cylinder 90. The proximal end portions 82, 92 may be sealed by glue or another chemical, or solvent bonding, or heat welded, or sonically welded together.

In one embodiment, the first proximal portion 82 of the first cylinder 80 is sealed to both the second proximal portion 92 of the second cylinder 90 and to the rear tip 50.

In one embodiment, the first cylinder 80 is formed independently of the second cylinder 90, then the first cylinder 80 is inserted into the second cylinder 90 with the void space 110 between the cylinders 80, 90, and the proximal end portions 82, 92 are bonded together. In this way, the gap distance D is formed by selecting a diameter of the first cylinder 80 to be less than a diameter of the second cylinder 90, and when nested together, the gap distance is one-half of the difference in the diameters. By way of example, when the diameter of the first cylinder 80 is selected to be 14 mm and the diameter of the second cylinder 90 is selected to be 20 mm, then the gap distance is 3 mm between the two nested cylinders. A pinhole of small opening may be provided in the outer, second cylinder 90 to allow trapped air to escape the inflatable bladder 60 as it is inflated. In one embodiment, less than an entirety of a perimeter of the first proximal portion 82 of the first cylinder 80 is sealed to the second proximal portion 92 of the second cylinder 90 to allow a path for the escape of trapped air.

The embodiments described above generally illustrate a prosthesis with two nested cylinders to provide the inflatable bladder with a multi-wall structure.

Embodiments described below provide a prosthesis having a bladder wall composed of multiple wall portions, which can be realized from a single inflatable bladder wall that is split into multiple thinner walls, or an inflatable bladder formed from a plurality of nested cylinders.

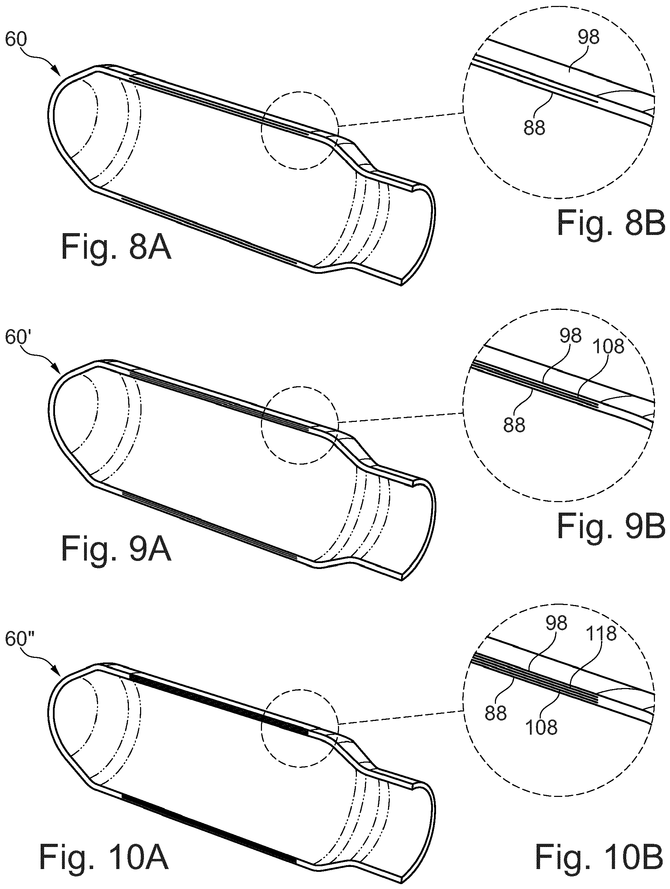

Fig. 8A and Fig. 8B are cross-sectional views of the embodiment of the inflatable bladder 60 having two separated walls 88, 98.

Fig. 9A and Fig. 9B are cross-sectional views of one embodiment of an inflatable bladder 60' having three separated walls 88, 98, and 108. The three-layered wall structure is realized by separating the middle wall 108 from each of its adjacent walls 88, 98. Alternatively, the middle wall is provided by a separate third cylinder 108, where the inflatable bladder 60' is provided by a pair of nested cylinders 80, 90 with a third cylinder 108 between the cylinders 80, 90.

Fig. 10A and Fig. 10B are cross-sectional views of one embodiment of an inflatable bladder 60" having four separated walls, including the inner wall 88, the outer wall 98, a first inner wall provided by a third cylinder 108 adjacent to the inner wall 88, and a second inner wall provided by a fourth cylinder 118 located between the first inner wall 108 and the outer wall 98. The inflatable bladder 60" is provided by a pair of nested cylinders 80, 90, with a third cylinder 108 adjacent to the first cylinder 80 and a fourth cylinder 118 between the third cylinder 108 and the second (or outer) cylinder 90. Alternatively, the inflatable bladder 60" is a cylinder having the four walls 88, 98, 108, 118 separated one from another along its mid-portion.

Thus, embodiments allow for an inflatable bladder formed to have a plurality of walls, each separated one from another, and so configured to move independently, and this feature increases the flexibility of the inflatable bladder when bent or buckled to provide reduced dog-ear effects and improved user comfort.

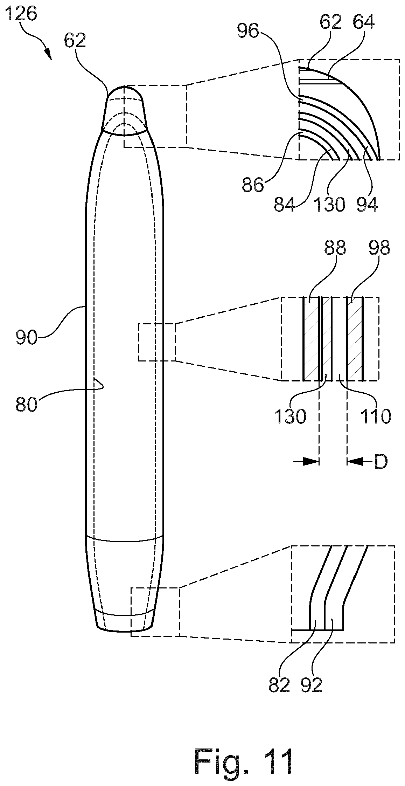

Fig. 11 is a series of partial cross-sectional views of one embodiment of an inflatable bladder 126 including a spacing material added to physically separate the inner cylinder from the outer cylinder. Adding a spacer material between the cylinders could aid in manufacturability, particularly if the spacer material is associated with or attached to the inner cylinder before the two cylinders are nested. A portion of the inflatable bladder 126 is sealed to the rear tip 50 (See Figs. 4-6) when assembling a penile prosthesis.

The inflatable bladder 126 includes the nested cylinders 80, 90 and a spacing material 130 inserted into the gap distance D and adapted to separate the second wall 98 and the first wall 88. The spacing material 130 prevents adhesion between the second wall 98 and the first wall 88 by reducing or preventing contact between the first cylinder 80 and the second cylinder 90 when the prosthesis 126 is uninflated. The spacing material 130 is placed between the two cylinders 80, 90 during assembly of the inflatable bladder 126, for example the spacing material 130 is inserted as the first cylinder 80 is nested with the second cylinder 90. Suitable spacing materials include a cylinder or a portion of a cylinder or a segment of a wall formed of a woven textile, a knitted textile, a wax, a foam, a film, a gel, or a silicone liquid deposited between the cylinders 80, 90.

In one embodiment, the spacing material 130 is a polymer layer that encourages separation between the two nested cylinders. One system of nested cylinders includes forming the cylinders of a urethane polymer system and selecting a spacer material 130 that encourages a separation between the two urethane cylinders. In one embodiment, the spacer material 130 is a silicone polymer system that has been measured to provide an effective separation between two urethane-based cylinders. In one example, the spacer material 130 was chosen to be a polytetrafluoroethylene (PTFE) system and measurements of the associated radial flexibility during bending indicates that the PTFE spacer material is less effective than the silicone polymer system spacer material at reducing dog-ears.

The spacer material 130 may be spray coated, roll coated, or dip coated onto the exterior surface of the first cylinder 80 (the inner cylinder).

In one embodiment, the spacing material 130 is a low adhesion surface coating applied to the first wall 88 and adapted to allow the first wall 88 to slide relative to the second wall 98. The spacing material 130 need not occupy the entire space 110 between the cylinders, 80, 90 but is included to ensure separation of at least the walls 88, 98 of the cylinders 80, 90, respectively.

One approach to fabrication of the inflatable bladder 126 includes inserting the first cylinder 80 within the second cylinder 90 and placing the spacing material 130 between the two cylinders 80, 90. The proximal portions 82, 92 are subsequently bonded together. It is acceptable to bond the spacing material 130 to the proximal portions 82, 92 when assembling the inflatable bladder 126. It is also acceptable to have the spacing material located distal to the proximal portions 82, 92 and located between the walls 88, 98. The spacing material 130 ensures that the first cylinder 80 is separate and free to move independently from the second cylinder 90 along the walls 88, 98 and along the distal end portions 84, 94.

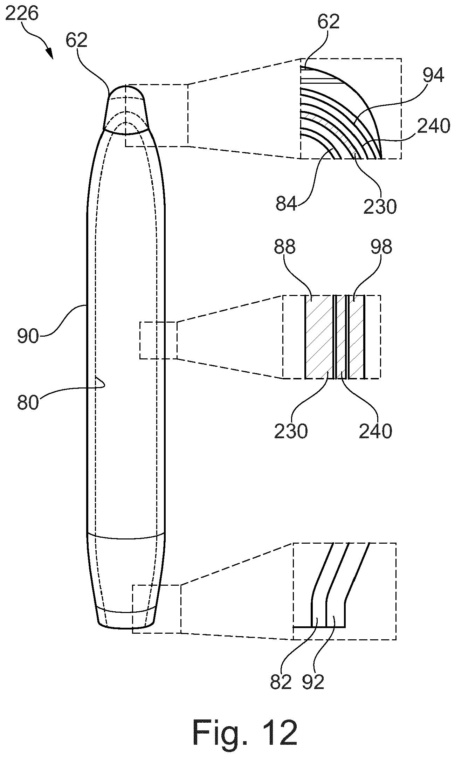

Fig. 12 is a series of partial cross-sectional views of one embodiment of an inflatable bladder 226 with a lubricating liquid between nested cylinders to encourage slip and independent movement between cylinders, which has been measured to aid in reducing dog-ears formed during bending of a deflated prosthesis. The inflatable bladder 226 includes the first cylinder 80 nested within the second cylinder 90, a polymer 230 coated onto an exterior of the first cylinder 80, and a liquid 240 located between the polymer 230 and the second cylinder 90. A portion of the inflatable bladder 226 is sealed to the rear tip 50 (See Figs. 4-6) when assembling a penile prosthesis

In one embodiment, each of the first cylinder 80 and the second cylinder 90 is formed of a first polymer, for example urethane, and the second polymer 230 is different from the first polymer, for example silicone. In one approach, the second polymer 230 is coated onto the first wall 88 of the first cylinder 80 and is adapted to separate the second wall 98 and the first wall 88. In one example, the first polymer for the cylinders 80, 90 is polyurethane and the second polymer is a polyolefin, a polyester, a different polyurethane, or a silicone polymer. In one embodiment, the liquid 240 located between the second polymer 230 and the second wall 98 of the second cylinder 90 is a silicone oil or a silicone dispersion. One suitable liquid 240 is a silicone dispersion available from NuSil (Avantor of Allentown, PA) identified as MED-361. The liquid acts as a lubricating layer to encourage separation between the cylinders 80, 90.

One approach to fabricating the prosthesis 226 includes molding or forming the first cylinder 80 and subsequently coating the polymer 230 onto its exterior surface, or coating more than one polymer onto its exterior surface while including the polymer 230 coating. The second cylinder 90 is formed independently. The first cylinder 80 with the polymer on its exterior surface is inserted into the second cylinder 90 and the liquid 240 is injected, for example by a syringe, into the space between polymer 230 on the first cylinder 80 and the wall 98 of the second cylinder 90. The proximal ends 82, 92 of the cylinders 80, 90, respectively, are bonded to form a prosthesis 226 with a closed and nested system of cylinders.

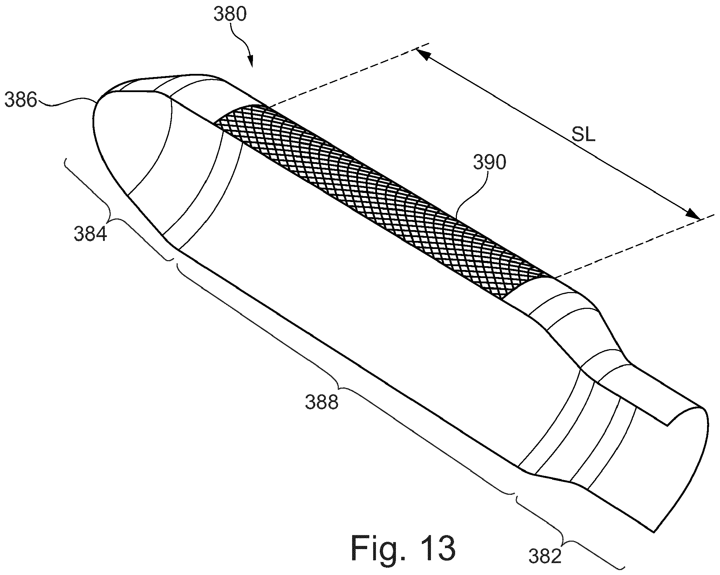

Fig. 13 is a perspective view of one embodiment of an interior cylinder 380 of a multicylinder nested prosthesis, with the interior cylinder 380 including separation layer in the form of an annular sleeve 390. The interior cylinder 380 includes a first proximal portion 382, a first distal end portion 384 that terminates in a first distal end 386, and a first wall 388 extending between the first proximal portion 382 and the first distal end portion 384.

One advantage of a selective separation layer like the annular sleeve 390 is that it allows options in both material selection and fabrication processes. For example, the separation layer could be the annular sleeve 390 positioned at a desired location along the cylinder 380, or the separation layer could be a sheet that is wrapped around the cylinder 380. In one embodiment, the separation layer 390 is provided as a heat-shrinkable or solvent-expandable sleeve, which could make manufacturing easier. In one embodiment, the separation layer 390 is two-ply or multi-ply film with successive wrappings. In this embodiment, the cylinder 380 is formed and a first ply of a multi-ply system is wrapped around an exterior surface of the cylinder 380; a coating is applied over the first ply in the system; and a subsequent ply is wrapped around the coated first ply. The multiple plies and one or more coatings provides improved slippage between the two plies. The film thickness for the cylinder 380 and the plies is selected to be in a range between 0.0127 mm to 0.0254 mm (0.0005 to 0.0010 inches) to reduce bulging in the nested cylinders of the prosthesis. Some materials naturally have low surface energy and so it may be desirable to apply a surface treatment like chemical, corona, or plasma etching on the outward facing surface so that the subsequent coatings are encouraged to wet the surface.

The annular sleeve 390 is wrapped or coupled to the first wall 388 of the first cylinder 380 and is adapted to separate the second wall (similar to the second wall 98 of the second cylinder 90 in Figs. 4-6) and the first wall 388 in a nested-cylinder prosthesis.

In one embodiment, the annular sleeve 390 has a sleeve length SL and a sleeve diameter SD, where the sleeve length SL is less than a length of the first wall 388 measured between the first proximal portion 382 and the first distal end portion 384 of the cylinder 380.

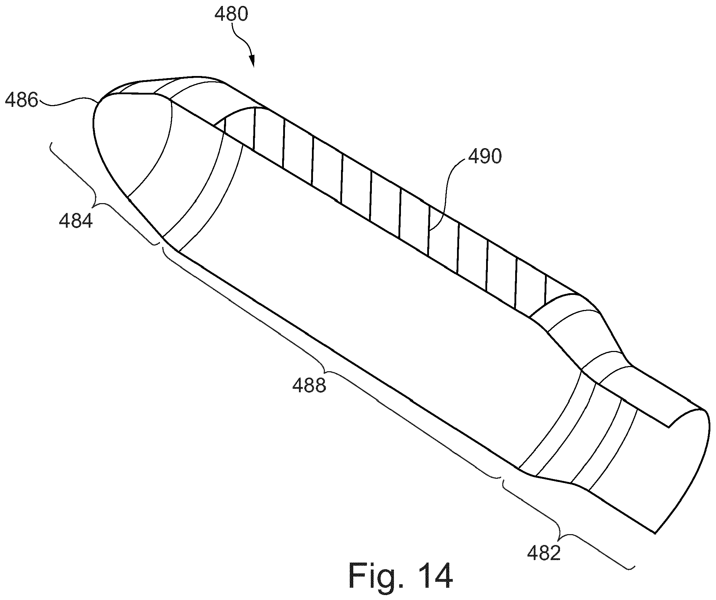

Fig. 14 is a perspective view of one embodiment of an interior cylinder 480 of a multicylinder nested prosthesis, with the interior cylinder 480 including separation layer in the form of a filament 490. The interior cylinder 480 includes a first proximal portion 482, a first distal end portion 484 that terminates in a first distal end 486, and a first wall 488 extending between the first proximal portion 482 and the first distal end portion 484. The filament 490 is wrapped multiple revolutions around a circumference of the first wall 488 of the first cylinder 480 and is adapted to separate the second wall (similar to the second wall 98 of the second cylinder 90 in Figs. 4-6) and the first wall 488 in a nested-cylinder prosthesis.

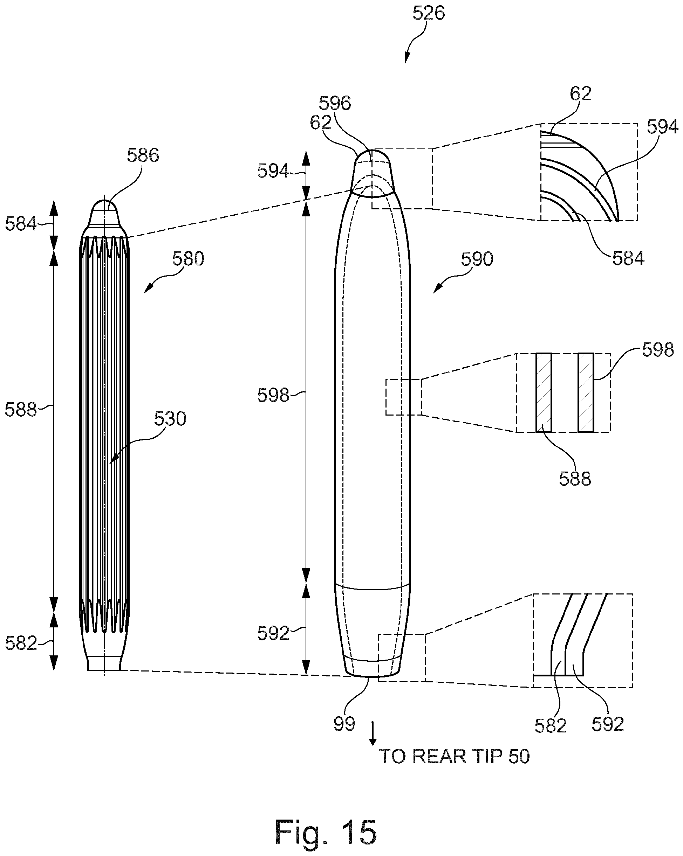

Fig. 15 is a view of one embodiment of an inflatable bladder 526. Fig. 15 provides an exploded view of a first cylinder 580 removed from a second cylinder 590 and includes a series of partial cross-sectional views along the inflatable bladder 526 illustrating a spacing between walls of the inflatable bladder 526.

The inflatable bladder 526 is a system of two nested cylinders 580, 590, where the first cylinder 580 includes a pattern of waves 530 or undulations 530 that provides a wavy-walled cylinder of troughs and peaks. The first cylinder 580 with the pattern of waves 530 is nested inside of the second cylinder 590, and this arrangement has been evaluated to improve flexibility and reduce the occurrence of dog-ears when this structure of an inflatable bladder is included in a prosthesis.

The first cylinder 580 includes a first proximal portion 582, a first distal end portion 584 that terminates in a first distal end 586, and a first wall 588 extending between the first proximal portion 582 and the first distal end portion 584. Likewise, the second cylinder 590 includes a first proximal portion 592, a first distal end portion 594 that terminates in a first distal end 596, and a first wall 598 extending between the first proximal portion 592 and the first distal end portion 594. The end tip 62 is connected to the second distal end portion 594 of the second cylinder 590. When the inflatable bladder 526 is assembled and connected to a rear tip 50 to form a penile prosthesis, the first cylinder 580 is nested within the second cylinder 590 and the proximal portions 582, 592 are joined together. At least the second proximal portion 592 of the second cylinder 590 is sealed to the rear tip 50 (See Figs. 4-6). When assembled, the first cylinder 580 is inflatable with liquid to inflate the prosthesis. Measurements show that the wavy first cylinder 580 reduces the presence of dog-ears when the deflated prosthesis is bent on its long axis.

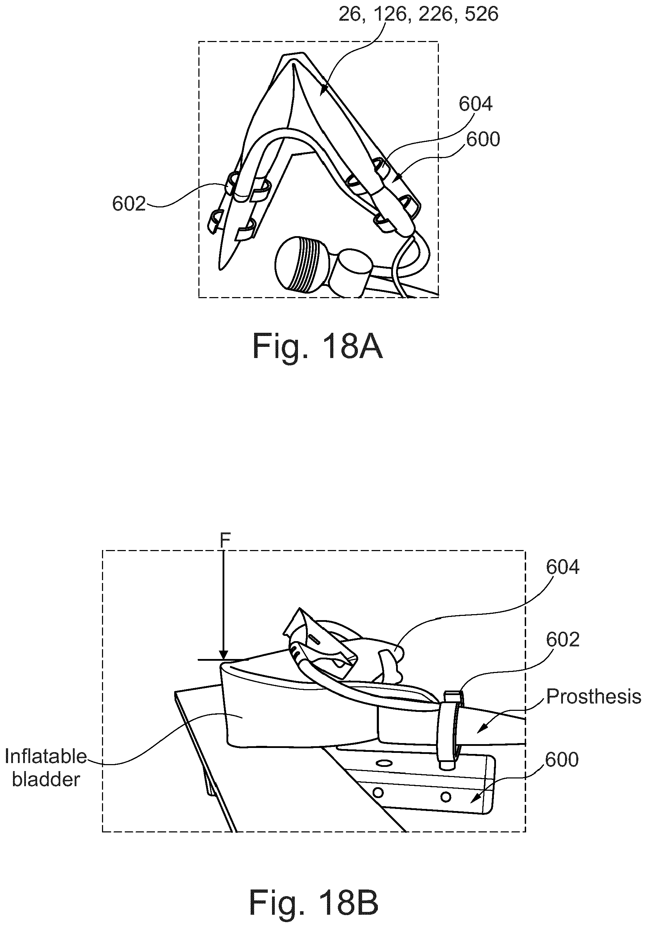

The undulations 530 on the wavy first cylinder 580 forms a spacing from the second cylinder 590 and has been measured to reduce the dog-ear buckling force applied to such a prosthesis. The dog-ear buckling force relates to an increase in flexibility when the prosthesis is first bent on its long axis (longitudinal bending), as would occur when a deflated cylinder becomes bent during use (see Figs. 2A and 18A), and then a transverse force is applied at the dog-ear (see Fig. 18B), which would be analogous to tissue pressing against the corners of the dog-ear during use. Dog-ear buckling is characterized by increasing the transverse force applied at a dog-ear corner until the dog-ear corner eventually buckles, and this measured value is referred to as the dog-ear buckling force. Consequently, the undulations on the wavy first cylinder 580 result in a desirable and measurable reduction in the occurrence of dog-ears.

The undulations 530 on the wavy first cylinder 580 provide an increased radius at the lateral corners where the opposed sides of the deflated cylinder come together when the deflated prothesis is bent on its long axis, thus reducing the presence of dog-ears. When the wavy first cylinder 580 is nested within the second cylinder 590, the result is an inflatable bladder that may be bent when deflated while having a reduced or eliminated presence of dog-ears.

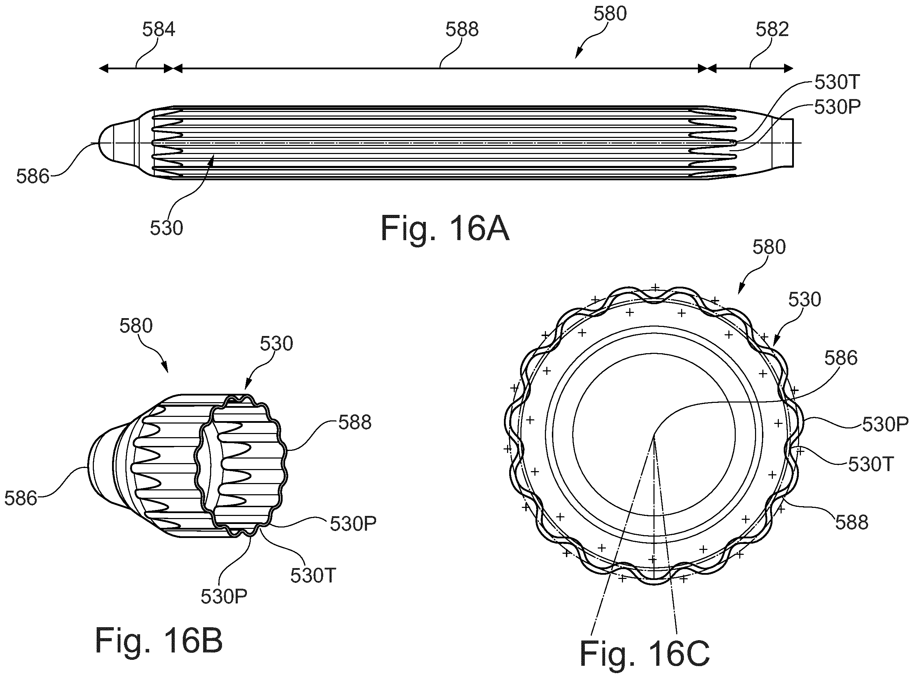

Fig. 16A is a side view, Fig. 16B is a perspective view of the distal end portion 584, and Fig. 16C is a distal end view of the distal end 586 of the first cylinder 580.

The undulations 530 on the wavy first cylinder 580 includes a series of troughs 530T and a series of peaks 530P, with each trough 530T located between two adjacent peaks 530P. The first wall 588 is formed to have troughs 530T aligned longitudinally along the first wall 588. The troughs 530T approximate a sine wave having a peak 530P formed by an exterior surface of the first wall 588 located between adjacent troughs 530T. In one embodiment, the troughs 530T are formed as a smooth and continuous curve in the wall 588 and the peaks 530P are formed of smooth and continuous curves, which associates with the sine curve familiar in trigonometry. In another embodiment, the undulations 530 could be triangular-shaped or rectangular-shaped, which associate with a discontinuous wave pattern or a pattern of square waves, as examples.

In one embodiment, the first cylinder 580 has a thickness (or thinness) for the wall 588 of about 0.2 mm and a length between the proximal end and the distal end 586 of about 139 mm. The pattern of undulations 530 extend along the wall 588 for about 110 mm. The pattern of undulations 530 includes about 15 troughs 530T around the circumference of the first cylinder 580, and each trough 530T has a depth of about 0.76 mm, thus the "amplitude" of the peak 530P is larger than the thickness of the wall 588, which is to say that the wall 588 itself is wavy or undulated. The selected size of a prosthesis depends upon the size of the dilated corpus cavemosum, so the length of the second cylinder 590 and the length of the first cylinder 580 vary. Approximate examples of prostheses have lengths from about 110 mm to 280 mm (this includes the length of the rear tip plus the length of the inflatable bladder), thus lengths for the second cylinder range from about 70 mm to 260 mm, with lengths of the shorter first cylinder 580 (the inner cylinder) ranging from about 65 mm to 255 mm, approximately.

Suitable wavy cylinders include a pattern of undulations 530 with fewer than 15 troughs 530T or more than 15 troughs 530T around the circumference of the first cylinder 580. The illustrated pattern of 15 troughs 530T is provided as an example only, and other patterns for the undulations 530 are possible and acceptable. As an example, a prosthesis for a larger penis would likely have a longer length, a larger diameter, and more troughs and peaks within the undulations 530.

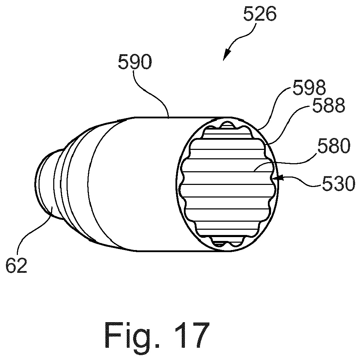

Fig. 17 is a perspective view of a section of a distal portion of an assembled inflatable bladder 526. The wavy first cylinder 580 is inside of the second cylinder 590. The sectional view of Fig. 17 is taken through the wall 598 of the second cylinder 590 and the wall 588 of the wavy first cylinder 580. The wavy undulations 530 provide a separation between the cylinders 580, 590 particularly along the walls 588, 598 and the distal end portions 584, 594. The nested and separated cylinder 580, 590 provide a penile prosthesis with improved bending flexibility, which reduces the presence of the dog-ears. The nested cylinders 580, 590 together to combine to offer good stiffness in the axial direction when inflated, which helps to provide an erection.

Fig. 18A is a top view and Fig. 18B is a perspective view of one of the penile prostheses 26, 126, 226, 526, or a prosthesis having an inflatable bladder including one of the interior cylinders 380 or 480, as described above inserted into a test fixture 600. The test fixture 600 includes brackets 602, 604 that couple to the prosthesis to maintain the inflatable bladder portion at a fixed angle. The test fixture 600 holds an inflatable bladder in a longitudinally bent configuration (Fig. 18A) to form a dog-eared corner. A force is applied to the dog-ear corner as shown in Fig. 18B. The measured force that causes the dog-ear formed in the inflatable bladder to buckle is recorded as the dog-ear buckling force.

The test fixture 600 allows evaluation of penile prosthesis to measure the force required to buckle the corner dog-ear formed in a deflated inflatable bladder. The lower the force measured to buckle the dog-ear corner of the deflated bladder, the lower the dog-ear buckling force, and the lower the severity of the dog-ear effect.

Fig. 18A shows longitudinal bending imposed on the inflatable bladder. The test fixture 600 holds a deflated inflatable bladder of the prosthesis at a fixed angle, in this case the angle is 60 degrees. A machine, for example a universal testing system available from INSTRON, Norwood, MA, is used to apply a force to the bent portion of bladder (the dog-eared corner), as shown in Fig. 18B. An increasing force F is applied to the bent inflatable bladder until the dog-ear corner of the bladder buckles, and the force measured at the time of buckling is recorded as the dog-ear buckling force. Generally, a dog-ear buckling force around 3.5 Newtons (or 0.8 lbf) is associated with inflatable bladders that form a dog-ear corner in the bladder. A lower dog-ear buckling force, for example less than 2 N (or .45 lbf), is associated with reduced or no formation of perceived dog-ears in a bent, deflated bladder.

Table A compares the buckling force of exemplary embodiments of Example C through Example F to the prior art inflatable bladders IB and IB2 of Figs. 1A-1C.

A user of a penile prosthesis can sense the presence of a dog-ear in a deflated bladder when the flaccid penis is bent, and the sensation is characterized as uncomfortable according to users. The amount that the bladder is deflated can vary depending on user conditions and the time and effort applied by the user to evacuate the inflatable bladder of liquid. For this reason, the dog-ear buckling force was measured for inflatable bladders across a range of deflation states where the bladder was allowed to retain some portion of liquid, or a "fill volume percent" of liquid, in a range from about 30% to about 50% relative to the volume in the inflated state. The dog-ear buckling force was measured three times and then averaged across varying fill volume percentages since it is unknown how much liquid stays in the system when a user deflates an inflatable bladder to the deflated, flaccid state.

Table A

| Example | Buckling Force (N) averaged across a range of Total Volume (%) from 30-50% | Buckling Force (lbf) averaged across a range of Total Volume (%) from 30-50% |
|---------|---------------------------------------------------------------------------|------------------------------------------------------------------------------|
| A       | 4.14                                                                      | 0.93                                                                         |

(continued)

| Example | Buckling Force (N) averaged across a range of Total Volume (%) from 30-50% | Buckling Force (lbf) averaged across a range of Total Volume (%) from 30-50% |
|---|---|---|
| B | 1.60 | 0.36 |
| C | 3.47 | 0.78 |
| D | 1.69 | 0.38 |
| E | 0.85 | 0.19 |
| F | 1.82 | 0.41 |

Example A - was an inflatable bladder IB formed of a single wall of urethane material.

Example B - was an inflatable bladder IB2 with a single wall thickness formed of a laminate of bonded layers of soft, low durometer silicone and a fabric.

Example C - was an inflatable bladder like inflatable bladder 126 having two nested cylinders including an interior cylinder with a silicone spacing material dip-coated to an exterior of the interior cylinder, as described above.

Example D - was an inflatable bladder like inflatable bladder 226 having two nested cylinders including an interior cylinder with a silicone spacing material dip-coated to an exterior of the interior cylinder and a lubricating liquid between the nested cylinders, as described above.

Example E - was an inflatable bladder like inflatable bladder 60 having two nested cylinders having two "stacked" and spaced apart walls, as described above.

Example F - was an inflatable bladder like inflatable bladder 526 having two nested cylinders including a first interior cylinder having a pattern of waves or undulations inserted into an exterior cylinder, as described above.

A buckling force of less than about 2.25 N (0.50 lbf) provides a reduced level of dog-ears. Example B has an acceptable level of dog-ears but is limited in inflation pressure and tensile stiffness, which limits the strength of the erection provided by such a prosthesis. In contrast, Examples D, E, and F provide a prosthesis with improved flexibility and reduced presence of dog-ears in the deflated state while also presenting with improved toughness and tensile strength during the inflated state, when compared to Example A and Example B.

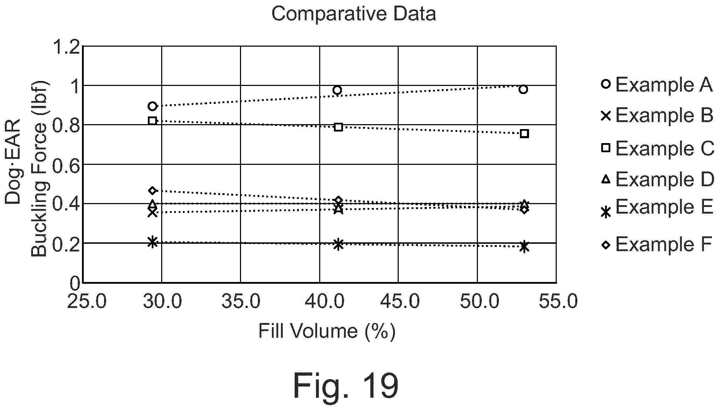

Fig. 19 is a graph of the same comparative data for Example A through Example F plotting the average dog-ear buckling force (in lbf) averaged over fill volume percentages ranging from about 30% to about 53%. The presence of dog-ears is lower for lower values for the averaged buckling force. Thus, Example E with an inflatable bladder like inflatable bladder 60 having two nested cylinders with spaced apart walls, has the lowest average dog-ear buckling force in the deflated state for the examples and the lowest presence of dog-ears while also having excellent tensile strength, girth, and qualitative erection when inflated.

An inflatable bladder of a penile prosthesis is described having a multi-layered wall structure with a wall spacing between the multi-layered walls to allow an interior distal wall end to move independently of an exterior distal wall end, which has been shown to reduce the dog-ear buckling force of an inflatable bladder in a flaccid state.

An inflatable bladder of a penile prosthesis is described having a multi-layered wall structure where the first distal end is spaced away from the second distal end by a gap distance allowing the first distal end to move independently of the second distal end.

An inflatable bladder of a penile prosthesis is described having a multi-layered wall structure where the first distal end is not connected to the second distal end allowing the first distal end to move independently of the second distal end.

Although specific embodiments have been illustrated and described herein, it will be appreciated by those of ordinary skill in the art that a variety of alternate and/or equivalent implementations may be substituted for the specific embodiments shown and described without departing from the scope of the present invention. This application is intended to cover any adaptations or variations of medical devices as discussed herein. Therefore, it is intended that this invention is limited only by its claims and their equivalents.

**Claims**

1.  A penile prosthesis (26, 60', 60", 126, 226, 526) comprising:

    a rear tip (50), with a lumen (70) formed inside of the rear tip (50), and tubing (72,32) connected to the rear tip (50) and communicating with the lumen (70); and

an inflatable bladder (60) coupled to the rear tip (50), the inflatable bladder (60) comprising:
a first cylinder (80) nested inside a second cylinder (90):

the first cylinder (80) comprising a first proximal portion (82), a first distal end portion (84) that terminates in a first distal end (86), and a first wall (88) extending between the first proximal portion (82) and the first distal end portion (84),
the second cylinder (90) comprising a second proximal portion (92), a second distal end portion (94) that terminates in a second distal end (96), and a second wall (98) extending between the second proximal portion (92) and the second distal end portion (94);

wherein the first proximal portion (82) of the first cylinder (80) is connected to the rear tip (50)
wherein an interior (100) of the first cylinder (80) provides the inflatable bladder with an inflation volume V communicating with the lumen (70) in the rear tip (50);
wherein the first distal end (86) is spaced from the second distal end (96) by a gap distance D allowing the first distal end (86) to move independently of the second distal end (96).

2. The penile prosthesis of claim 1, wherein the first proximal portion (82) of the first cylinder (80) is connected to the second proximal portion (92) of the second cylinder (90), and the second proximal portion (92) of the second cylinder (90) is connected to the rear tip (50).

3. The penile prosthesis of claim 1, wherein the second wall (98) is spaced away from the first wall (88) by the gap distance D allowing the first distal end (86) to move independently of the second distal end (96).

4. The penile prosthesis of claim 1, wherein less than an entirety of a perimeter of the first proximal portion (82) of the first cylinder (80) is sealed to the second proximal portion (92) of the second cylinder (90).

5. The penile prosthesis of claim 1, further comprising a third cylinder (108) nested between the first cylinder (80) and the second cylinder (90), with a proximal portion of the third cylinder (108) sealed between the first proximal portion (82) of the first cylinder (80) and the second proximal portion (92) of the second cylinder (90).

6. The penile prosthesis of claim 1, further comprising a spacing material (130) inserted into the gap distance D and adapted to separate the second wall (98) and the first wall (88).

7. The penile prosthesis of claim 6, wherein the spacing material prevents adhesion between the second wall (98) and the first wall (88).

8. The penile prosthesis of claim 6, wherein the spacing material (130) is selected from the group consisting of a woven textile, a knitted textile, a wax, a foam, a film, a gel, and a silicone liquid.

9. The penile prosthesis of claim 1, further comprising a low adhesion surface coating applied to the first wall (88) .

10. The penile prosthesis of claim 1, wherein each of the first cylinder (80) and the second cylinder (90) is formed of a first polymer, and further comprising a second polymer (230) different from the first polymer, with the second polymer (230) coated onto the first wall (88) of the first cylinder (80) and adapted to separate the second wall (98) and the first wall (88).

11. The penile prosthesis of claim 10, further comprising a liquid (240) located between the second polymer (230) coated onto the first wall (88) of the first cylinder (80) and the second wall (98) of the second cylinder (90).

12. The penile prosthesis of claim 1, further comprising an annular sleeve (390) coupled to the first wall (88) of the first cylinder (80) and adapted to separate the second wall (98) and the first wall (88).

13. The penile prosthesis of claim 12, wherein the annular sleeve (390) has a sleeve length SL and a sleeve diameter SD, where the sleeve length SL is less than a length of the first wall (88) measured between the first proximal portion (82) and the first distal end portion (84).

14. The penile prosthesis of claim 1, further comprising a filament (490) wrapped multiple revolutions around a circumference of the first wall (488) of the first cylinder (480) and adapted to separate the second wall (98) and the first wall (488).

15. The penile prosthesis of claim 1, wherein the first wall (588) of the first cylinder (580) comprises a series of troughs (530T) formed into the first wall (588) and aligned longitudinally along the first wall (588).

16. The penile prosthesis of claim 15, wherein the series of troughs (530T) approximate a sine wave having a peak (530P) formed by an exterior surface of the first wall (588) located between adjacent troughs (530T).

PRIOR ART

PI

IB

IB

IB

## Fig. 1A

IB

W

## Fig. 1B

DE

Fig. 2B

PRIOR ART

Fig. 2A

Fig. 3

EP 4 140 450 A1

Fig. 4

Fig. 5

EP 4 140 450 A1

Fig. 6

60

62

96

62

64

D

86

84 110 94

88 98

90

80

80

100 110

80

90

D

82 92

Fig. 7

Fig. 8A

Fig. 8B

Fig. 9A

Fig. 9B

Fig. 10A

Fig. 10B

Fig. 11

Fig. 12

Fig. 13

Fig. 14

Fig. 15

584 | 588 | 580 | 582
530T
530P
586
530

Fig. 16A

580
530
588
586
530P
530T
530P

Fig. 16B

580
530
586
530P
530T
588

Fig. 16C

EP 4 140 450 A1

Fig. 17

26, 126, 226, 526

604

600

602

## Fig. 18A

F

604

602

Prosthesis

Inflatable
bladder

600

## Fig. 18B

Comparative Data

Fig. 19

EP 4 140 450 A1

Europäisches
Patentamt

European
Patent Office

Office européen
des brevets

# EUROPEAN SEARCH REPORT

**Application Number**

EP 22 18 9851

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/138302 A1 (AMS RES CORP [US]) 17 September 2015 (2015-09-17) | 1-3,5-13 | INV. A61F2/26 |
| Y | * paragraphs [0006], [0024] - [0043]; | 15,16 | |
| A | figures 1,3,6 * | 14 | |
| | ----- | | |
| X | US 4 881 530 A (TRICK ROBERT E [US]) 21 November 1989 (1989-11-21) * the whole document * | 1-3,6-9 | |
| | ----- | | |
| X | US 2009/124851 A1 (KUYAVA CHARLES C [US]) 14 May 2009 (2009-05-14) | 1,3,4 | |
| Y | * paragraphs [0005] - [0007], [0019] - [0032]; figures 1-4D * | 15,16 | |
| | ----- | | |

**TECHNICAL FIELDS SEARCHED (IPC)**

A61F

The present search report has been drawn up for all claims

| Place of search | Date of completion of the search | Examiner |
|---|---|---|
| Munich | 17 January 2023 | Steiner, Bronwen |

EPO FORM 1503 03.82 (P04C01)

## ANNEX TO THE EUROPEAN SEARCH REPORT
## ON EUROPEAN PATENT APPLICATION NO.

EP 22 18 9851

This annex lists the patent family members relating to the patent documents cited in the above-mentioned European search report.
The members are as contained in the European Patent Office EDP file on
The European Patent Office is in no way liable for these particulars which are merely given for the purpose of information.

17-01-2023

| Patent document cited in search report | | Publication date | Patent family member(s) | Publication date |
|---|---|---|---|---|
| WO 2015138302 | A1 | 17-09-2015 | NONE | |
| US 4881530 | A | 21-11-1989 | NONE | |
| US 2009124851 | A1 | 14-05-2009 | NONE | |

EPO FORM P0459

For more details about this annex : see Official Journal of the European Patent Office, No. 12/82